# FASCICULE DE BREVET EUROPEEN

(11) **EP 3 727 596 B1**
(45) Date de publication et mention de la délivrance du brevet: **03.09.2025**
(21) Numéro de dépôt: 18842782.7
(22) Date de dépôt: 19.12.2018
(51) Int. Cl.: A61Q 1/10, A61K 8/06, A61K 8/36, A61K 8/37, A61K 8/41, A61K 8/81, A61K 8/92

(54) **COMPOSITION DE MASCARA CONTENANT UN ESTER DE TÉTRAHYDROXYPROPYLÉTHYLÈNE DIAMINE**
MASCARA-ZUSAMMENSETZUNG MIT EINEM TETRAHYDROXYPROPYLETHYLENDIAMINESTER
MASCARA COMPOSITION CONTAINING A TETRAHYDROXYPROPYL ETHYLENEDIAMINE ESTER

(30) Priorité: 21.12.2017 FR 1762856
(43) Date de publication de la demande: 28.10.2020
(73) Titulaire: LVMH Recherche, 45800 Saint Jean de Braye (FR)
(72) Inventeur: PAYS, Karl, 94410 SAINT MAURICE (FR); BICHON, Yohann, 94700 MAISONS-ALFORT (FR)
(74) Mandataire: Cabinet Beau de Loménie
(86) Numéro de dépôt international: PCT/FR2018/053429
(87) Numéro de publication internationale: WO 2019/122727

(56) Documents cités:
- EP-A1- 0 663 202
- EP-A1- 0 928 607
- EP-A1- 3 228 304
- EP-A2- 0 023 978
- EP-B1- 0 928 607
- WO-A1-2015/177113
- CA-A1- 2 949 660
- US-A- 5 747 013
- US-A1- 2003 224 025
- US-A1- 2006 165 620
- DATABASE GNPD [online] MINTEL; May 2016 (2016-05-01), "Tinted Dual Brow", XP002780794, Database accession no. 3974221

## Description

La présente demande se rapporte au domaine du maquillage ou du soin des cils, notamment au domaine des mascaras crèmes et concerne plus spécifiquement une composition cosmétique applicable sur les fibres kératiniques.

L'invention concerne plus précisément un mascara contenant une quantité élevée de cires émulsionnées dans une phase aqueuse avec un ester d'acide gras et de tétrahydroxypropyl éthylène diamine (THPE).

### ETAT DE LA TECHNIQUE

Les mascaras cires-dans-eau contenant des esters de triéthanolamine (TEA) comme le stéarate de triéthanolamine sont reconnus pour leur qualité d'application douce, crémeuse et onctueuse. Les esters de triéthanolamine sont formés au cours du procédé de préparation du mascara par réaction d'un acide ou d'un mélange d'acides gras avec la triéthanolamine.

Cependant, la TEA entraine un risque élevé de formation de nitrosamines au cours du stockage et de l'utilisation du mascara conservé dans un conditionnement. La TEA peut en effet réagir avec les composants du conditionnement pour dégager des nitrosamines telles que la NDELA (N-nitrosodiethanolamine), si bien que son utilisation dans de nombreux produits est réglementée. Les produits cosmétiques, par exemple, doivent avoir une teneur en NDELA inférieure à 10 ppb pour respecter la réglementation des principaux pays européens.

Plusieurs propositions de substitution partielle ou totale de la TEA par d'autres amines hydroxylées pour former des esters d'acides gras ont été développées, mais les mascaras obtenus présentent des inconvénients.

C'est le cas notamment des mascaras cires-dans-eau contenant des esters d'aminométhyl propanediol (AMPD) qui sont moins crémeux et plus secs à l'application lorsque la TEA est totalement remplacée par l'AMPD. De telles formules sont par exemple décrites dans le document EP 3 228 304.

D'autres amines comme la trométhamine ont également été évaluées, mais les essais de substitution de la TEA par ces amines n'ont pas permis de la supprimer totalement des formules tout en conservant un résultat maquillage satisfaisant.

Les documents US 2003/224025 et US 2006/165620 décrivent des mascaras waterproof cires-dans-eau comprenant de l'acide stéarique et 1,3% en masse de tétrahydroxypropyl éthylène diamine.

Le besoin de disposer de mascaras crèmes en émulsion cires-dans-eau contenant des esters d'acides gras qui ne sont pas susceptibles de dégager des nitrosamines au cours de leur durée de vie, et qui ont une texture onctueuse à l'application persiste donc.

Plus précisément, on recherche un mascara dépourvu d'esters de triéthanolamine qui soient dotées de qualités d'application au moins équivalentes à celles des mascaras en contenant, et qui ne souffrent pas des désavantages de ceux qui ont été développés par substitution de la triéthanolamine.

Les inventeurs ont trouvé de façon surprenante, que les esters d'acides gras et de tétrahydroxypropyl éthylène diamine formulés dans des émulsions cires-dans-eau permettent d'obtenir des produits onctueux et crémeux, et agréables à utiliser sans risque de dégagement des nitrosamines.

Les esters d'acides gras et de tétrahydroxypropyl éthylène diamine peuvent substituer totalement les esters de triethanolamine dans les architectures existantes tout en conservant les mêmes propriétés, voire même en améliorant certaines d'entre elles.

Il a notamment été découvert que les esters d'acides gras et de tétrahydroxypropyl éthylène diamine permettent de proposer des nouvelles textures de mascaras, originales par leur sensorialité, leurs qualités d'application et leurs résultats maquillage.

La texture peut être plus fluide ce qui rend l'application plus glissante et plus agréable. Bien que l'émulsion soit plus fluide, la charge des cils reste équivalente, ce qui est très surprenant.

La formule peut également être plus adhérente ce qui améliore la tenue du produit sur les cils au cours du temps.

Enfin, la composition permet d'obtenir des teintes plus profondes, notamment des noirs plus intenses.

La tétrahydroxypropyl éthylène diamine a déjà été incorporée dans des compositions cosmétiques en faible concentration pour remplir la fonction d'ajustement du pH. Mais, dans ces compositions, la tétrahydroxypropyl éthylène diamine ne peuvent pas former les quantités significatives d'esters nécessaires dans les mascaras en émulsion pour stabiliser les cires dans une phase aqueuse.

### DESCRIPTION DE L'INVENTION

L'invention a donc pour objet une composition cosmétique de soin ou des maquillage des cils ou des sourcils comprenant des cires en émulsion dans une phase aqueuse et au moins un ester obtenu par réaction d'un acide gras linéaire et saturé comprenant de 16 à 20 atomes de carbone, et de tétrahydroxypropyl éthylène diamine, l'acide gras représentant de 2,0 à 8,0% en masse et l'amine représentant de 3,0 à 8,0% en masse, et le ratio molaire entre l'acide gras et l'amine étant compris entre 0,9 et 5. L'acide gras représente de 2,0 à 8,0% en masse, préférentiellement de 2,5 à 5,0% en masse, et plus préférentiellement de 4,0 à 6,0% en masse, par rapport à la masse de la composition.

L'amine représente de 3,0 à 8,0% en masse, préférentiellement de 3,0 à 7,0% en masse, plus préférentiellement de 3,0 à 6,0% en masse, et encore plus préférentiellement de 3,0 à 4,5% en masse, par rapport à la masse de la composition.

Le ratio molaire entre l'acide gras linéaire et saturé et la tétrahydroxypropyl éthylène diamine est de préférence compris entre 0,9 et 4,0, de préférence entre 1,0 et 3,0, et de préférence encore 1,1 et 2,0. On préfère utiliser l'acide gras en excès de manière à ce qu'il contribue à la structuration de la phase grasse.

Dans la présente description, les pourcentages en masse sont exprimés par rapport à la masse de l'émulsion sauf mention contraire.

La composition comprend de préférence moins de 1% en masse d'un composé ou d'un mélange de composés choisis parmi un ester de triéthanolamine, un ester d'aminométhyl propanediol, et un ester de trométhamine, qui sont susceptibles de nuire aux performances du mascara.

L'invention décrit également une composition cosmétique de soin ou de maquillage des cils ou des sourcils sous la forme d'une émulsion cires-dans-eau comprenant une quantité de composés portant une fonction amine susceptible de se décomposer en nitrosamines en une quantité inférieure à 10 ppb, caractérisée en ce qu'elle comprend un ester d'acide gras linéaire et saturé ayant de 16 à 20 atomes de carbone et de la tétrahydroxypropyl éthylène diamine, ledit ester étant en une quantité suffisante pour obtenir une viscosité dont la valeur est inférieure d'au moins 20% à la viscosité d'une composition identique comprenant un ester d'acide gras et d'une amine choisie parmi la triéthanolamine, la trométhamine et l'aminopropane diol.

La composition est avantageusement dépourvue d'esters d'un acide gras linéaire et saturé avec une amine choisie parmi la triéthanolamine, le l'aminopropanediol et de la trométhamine.

L'acide gras linéaire et saturé peut être l'acide stéarique, l'acide palmitique ou l'acide béhénique. Selon un mode de réalisation, on utilise un mélange d'acide stéarique et d'acide palmitique, par exemple dans un ratio massique compris entre 80/20 et 20/80, et avantageusement dans le ratio massique 50/50. Un mode de réalisation particulier comprend le mélange équipondéral d'acide stéarique et l'acide palmitique.

La tétrahydroxypropyl éthylène diamine a comme nomenclature IUPAC N,N,N',N'-Tetrakis(2-hydroxypropyl)ethylenediamine et comme dénomination INCI TETRAHYDROXYPROPYL ETHYLENEDIAMINE. Elle est vendue notamment sous les marques NEUTROL^{®} TE, LUTROPUR^{®} Q75 de BASF, et ADEKA CARPOL^{®} MD100 de ADEKA.

La quantité de l'ester d'acide gras et de tétrahydroxypropyl éthylène diamine est de préférence suffisante pour obtenir un pourcentage de diminution de la viscosité du mascara supérieure à une valeur choisie dans le groupe constitué de 10%, 15%, 20%, 25%, 30%, 35% et 40%, par rapport à un mascara de composition identique dans lequel l'ingrédient tétrahydroxypropyl éthylène diamine utilisé pour fabriquer le mascara est remplacé par la triéthanolamine.

La composition selon l'invention présente une texture fluide: sa viscosité est de préférence inférieure à 250 000 mPa.s, par exemple comprise entre 150 000 mPa.s et 230 000 mPa.s, de préférence entre 140 000 mPa.s et 210 000 mPa.s. La viscosité de la composition peut être mesurée à 25 °C à l'aide d'un Rhéomat^{®} 180 (Société LAMY) équipé d'un mobile MS-R1, MS-R2, MS-R3, MS-R4 ou MS-R5 choisi en fonction de la consistance de la composition, tournant à une vitesse de rotation de 200 t.min⁻¹. La mesure est prise après 10 min de rotation.

L'ester d'acide gras et de THPE remplit la fonction de tensio-actif anionique de HLB supérieur à 8. Dans la présente invention, les valeurs de HLB sont celles de Griffin à 25°C.

On peut éventuellement lui associer au moins un tensio-actif non ionique dont le HLB est inférieur à 8, notamment lorsque la quantité de cires dans la composition est supérieure à 20% en masse. Ce tensio-actif peut être choisi dans le groupe constitué par :
- les esters et éthers d'oses ;
- les esters d'acides gras, notamment en C8-C24, et de préférence en C16-C22, et de polyol, notamment de glycérol ou de sorbitol, de préférence de glycérol, comme le stéarate de glycéryle;
- les alcools oxyalkylénés comportant moins de 10 motifs oxyalkylénés mieux moins de 5 motifs oxyalkylénés, en particulier oxyéthylénés et/ou oxypropylénés.

Un alcool oxyéthyléné comprenant moins de 5 motifs oxyalkylénés peut être le composé de nom INCI STEARETH-2.

La composition peut également contenir un mélange de tensio-actifs comprenant l'ester d'acide gras et de THPE, au moins un tensio-actif non ionique dont le HLB est inférieur à 8 tel que décrit précédemment et au moins un tensio-actif non ionique de HLB supérieur à 8. Cette association est particulièrement intéressante lorsque le taux de cires dans la composition est supérieur à 30% en masse.

Ce dernier peut être choisi parmi :
- les alcools gras comprenant de 8 à 24 atomes de carbones (tels que l'alcool stéarylique) qui comprennent 20 motifs oxyéthylène (de nom INCI STEARETH-20, le composé de nom INCI CETEARETH-30 et le composé de nom INCI PARETH-7),
- le monostéarate de PEG-40,
- les esters d'acide gras comprenant 30 motifs oxyéthylène, tels que le stéarate de glycéryle polyoxyéthyléné comprenant 30 motifs oxyéthylène, l'oléate de glycéryle polyoxyéthyléné comprenant 30 motifs oxyéthylène, le cocoate de glycéryle polyoxyéthyléné comprenant 30 motifs oxyéthylène, l'isostéarate de glycéryle polyoxyéthyléné comprenant 30 motifs oxyéthylène, le laurate de glycéryle polyoxyéthyléné comprenant 30 motifs oxyéthylène, et
- les esters de sorbitane polyoxyéthylénés, tels que le polysorbate 60.

La composition peut contenir un tensio-actif anionique ayant une valeur de HLB supérieure ou égale à 8, tel que les alkylphosphates et les alkylsulfates. Dans un mode de réalisation particulier de l'invention, la composition en est dépourvue.

Les tensioactifs utilisés préférentiellement dans la composition selon l'invention sont choisis par exemple parmi les éthers oxyéthylénés et/ou oxypropylénés, les esters d'acide gras et de polyéthylène glycol, les polycondensats polyéthylène glycol/polypropylène glycol, les esters d'acides gras et de glycérol, tels que le stéarate de glycéryle, les esters d'acides gras et de sorbitol, les esters d'acides gras et de sorbitan, les esters de sucrose et les éthers de sucrose.

Selon un mode de réalisation particulier, la composition contient une quantité de cire(s) élevée, de préférence comprise entre 15 et 25% en masse.

Ces compositions particulières permettent avantageusement de déposer beaucoup de produit dès le premier passage de la brosse sur les cils, pour mieux gainer les cils, apporter un volume immédiatement perceptible par l'utilisateur, et augmenter la tenue du maquillage au cours du temps.

Or, les émulsions cires-dans-eau concentrées en cires de l'art antérieur peuvent manquer de fluidité, si bien que la quantité de produit déposée sur les cils devient trop importante et que l'enrobage des cils perd en homogénéité.

L'introduction d'ester d'acides gras linéaires et de tétrahydroxypropyl éthylène diamine dans des émulsions cires-dans-eau contenant des quantités importantes de composés solides tels que les cires, est donc particulièrement avantageuse, car elle permet de contre-balancer l'augmentation de consistance apportés par ces composés en abaissant la viscosité de façon très significative. Dans ce type d'architecture, la composition de l'invention permet donc de façon tout à fait avantageuse, de proposer des mascaras crèmes apportant un volume immédiat, un allongement et une tenue dans le temps très performants, après une application lisse et homogène du produit.

On pourra utiliser toute cire connue par l'homme du métier de la cosmétique. La cire peut être par exemple choisie parmi :
- les cires alcanes dites également apolaires choisies parmi les cires issues de la distillation du pétrole comme les cires microcristallines et certaines cires de paraffine ; les cires de synthèse obtenues par polymérisation du méthylène, de l'éthylène ou du propylène comme la cire de polyméthylène, la cire de polyéthylène, la cire de polypropylène, la cire de copolymère éthylène/propylène et la cire de copolymère ethylène/hexène ; les cires de Fischer Tropsch ; la cire d'ozokérite ; et la cérésine obtenue par raffinage de la précédente,
- les cires de silicone, et
- les cires qui ne sont ni siliconées, ni alcanes dites également polaires, telles que par exemple la cire d'abeille, la cire de son de riz, la cire de Carnauba, la cire de Candellila, la cire d'Ouricurry, la cire du Japon, la cire de Berry, la cire de Sumac, la cire de Montan, la cire d'Alfa, la cire de fibres de liège, la cire de canne à sucre, la cire d'orange, la cire de citron, la cire de laurier, les cires obtenues par hydrogénation d'huiles animales ou végétales ayant des chaînes grasses, linéaires ou ramifiées, en C8-C32, telles que l'huile de jojoba, l'huile de tournesol, l'huile de ricin, l'huile de coprah, l'huile de lanoline, l'huile d'olive estérifiée avec l'alcool stéarylique, l'huile de ricin estérifiée avec l'alcool cétylique.

Selon un mode de réalisation de l'invention, les cires représentent de 10 à 35% en masse, de préférence de 15% à 35% en masse, de préférence encore de 18% à 28% en masse, par rapport à la masse de la composition.

Dans un mode de réalisation particulier, la composition contient un mélange de cire apolaire et de cire polaire.

Dans un autre mode de réalisation, la composition contient 10 à 20% en masse d'une cire dont le point de fusion est supérieur ou égal à 70°C. Le point de fusion pourra être mesuré par toute méthode connue de l'homme du métier. On entend notamment par « température de fusion ou point de fusion » d'un composé, au sens de la présente invention, l'une des grandeurs suivantes : le point de fusion correspondant au début de la fusion de ce composé ou la température correspondant au pic endothermique du composé, ces grandeurs pouvant être mesurées par D.S.C. (Differential Scanning Calorimetry) selon la norme ISO 11357-3.

Une cire dont le point de fusion est supérieur ou égal à 70°C utilisée dans le cadre de l'invention peut être une cire de paraffine dont le point de fusion va de 62°C à 65°C, une cire de candellila, une cire d'abeille ou une cire de carnauba dont le point de fusion va de 78 à 85°C. Le point de fusion varie en fonction du procédé de fabrication de la cire et de la méthode utilisée pour le mesurer.

La composition peut contenir en particulier le mélange d'au moins une cire dont le point de fusion est supérieur ou égal à 70°C et d'au moins une cire dont le point de fusion est inférieur à 70°C, l'une des cires étant une cire apolaire et l'autre étant une cire polaire.

La composition peut contenir au moins un polymère qui remplit la fonction d'agent de texture, de filmogène ou de gélifiant. Le polymère peut notamment être destiné à augmenter le gainage des cils, l'allongement des cils et/ou leur recourbement.

La quantité de polymère peut aller de 2 à 20 %, de préférence de 3 à 10 % en masse par rapport à la masse de la composition.

Parmi les polymères envisageables figurent le polybutène, l'éthylcellulose et les copolymères de la vinylpyrolidone (VP) tels que la VP/eicosène ou la VP/hexadécène, le polydécène hydrogéné, les huiles siliconées non volatiles, notamment les phényltriméthicones, et leurs mélanges.

Parmi les polymères, on pourra encore citer les polymères de cellulose tels que l'hydroxyéthylcellulose, les polymères vinyliques comme les polyvinylpyrrolidones, les gommes arabiques, la gomme de guar, les dérivés du xanthane, les dispersions aqueuses de copolymères acrylate, et leurs mélanges.

La quantité totale de polymères et de cires dans la composition est de préférence comprise entre 15% et 35% de la composition.

La composition de mascara comprend des pigments représentant de 5 à 20% en poids, par exemple de 8 à 15% en masse de la composition.

Les pigments sont de préférence choisis parmi le dioxyde de titane, les oxydes de zinc, les oxydes de fer (noir, jaune ou rouge), le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques. On peut également citer les pigments réfléchissants et les pigments nacrés.

La phase liquide représente par exemple de 30% à 60% en masse, de préférence de 40% à 55% en masse, par rapport à la masse de la composition de mascara.

La phase liquide peut contenir de l'eau, des huiles volatiles et des polyols tels que le butylène glycol. Les huiles peuvent être choisies parmi les diméthicones volatiles, les cyclométhicones, l'isododécane, l'isohexadécane ou l'isodécane.

Selon un mode de mise en œuvre de l'invention, la phase liquide représente de 40% à 55% en poids, les cires représentent de 20% à 25% en poids, et les pigments représentent de 8 à 12% en poids.

Les compositions de l'invention peuvent contenir outre les ingrédients décrits précédemment, des adjuvants habituels dans le domaine de la formulation des mascaras, tels que des gélifiants hydrophiles ou lipophiles, des actifs, des huiles non volatiles, des composés gras sous forme de pâte, des conservateurs, des antioxydants, des ajusteurs de pH et des parfums.

Les huiles non volatiles peuvent être choisies parmi l'huile de paraffine, l'isononanoate d'isotridécyle, le néopentanoate d'isostéaryle, le néopentanoate de tridécyle, l'octanoate de cétyle. On préfère utiliser des huiles non volatiles d'origine végétale, comme l'huile de ricin, l'huile d'argan ou l'huile d'amande, qui peuvent également servir d'actif.

La description des ingrédients entrant dans la composition de l'invention peut être complétée du contenu de la demande FR 2 996 767.

L'invention est illustrée par les exemples suivants.

### Exemple 1, Exemple 2 comparatif et Exemple 3 comparatif:

Trois formules de mascaras ont été préparées en utilisant les ingrédients dans les proportions indiquées dans le Tableau 1 ci-dessous.

**TABLEAU 1 - Composition des mascaras**

| Phase | Ingrédients (Dénomination INCI) | Exemple 1 | Exemple 2 comparatif | Exemple 3 comparatif |
|---|---|---|---|---|
| A1 | WATER | Qsp 100 | Qsp 100 | Qsp 100 |
| A1 | PRESERVATIVES | 2,90 | 2,90 | 2,90 |
| A1 | TRIETHANOLAMINE | - | 2,00 | - |
| A1 | AMINOMETHYL PROPANEDIOL (AMPD ULTRA PC) | - | - | 1,40 |
| A1 | TETRAHYDROXYPROPYL ETHYLENEDIAMINE (NEUTROL TE) | 3,90 | - | - |
| A2 | HYDROXYETHYLCELLULOSE | 0,60 | 0,60 | 0,60 |
| A2 | ACACIA SENEGAL GUM | 2,00 | 2,00 | 2,00 |
| A2 | PVP (PVP K30) | 0,50 | 0,50 | 0,50 |
| B1 | STEARIC ACID | 2,00 | 2,00 | 2,00 |
| B1 | PALMITIC ACID | 2,00 | 2,00 | 2,00 |
| B1 | VP/EICOSENE COPOLYMER (ANTARON V 220 F) | 2,00 | 2,00 | 2,00 |
| B1 | POLYBUTENE (POLYBUTENE M 2000) | 2,00 | 2,00 | 2,00 |
| B1 | SYNTHETIC BEESWAX(KESTER WAX K 82P) | 12,00 | 12,00 | 12,00 |
| B1 | GLYCERYL STEARATE (CUTINA GMS-V) | 4,00 | 4,00 | 4,00 |
| B1 | CERA CARNAUBA | 4,00 | 4,00 | 4,00 |
| B1 | PARAFFIN | 10,00 | 10,00 | 10,00 |
| B2 | BLACK IRON OXIDE | 10,00 | 10,00 | 10,00 |

Afin d'effectuer une comparaison fiable, la quantité totale en acide stéarique et en acide palmitique a été maintenue constante pour les deux essais, et la quantité de THPE a été ajustée pour obtenir un ratio molaire THPE/(acides gras) égal.

### Procédé de préparation :

Préparer la phase aqueuse A1, mettre sous agitation STARO sous bain marie à 85°C.

Ajouter les gélifiants de phase aqueuse A2 dans A1 sous agitation.

Préparer la phase grasse B1, mettre sous bain marie à 85°C et sous agitation STARO.

Une fois la phase B1 fondue, ajouter le mélange des pigments et descharges B2 dans B1 sous agitation, et disperser pendant 30 min.

Emulsionner A dans B sous agitation forte au STARO à 85°C pendant 5 min puis retirer le bain-marie.

Laisser refroidir en diminuant progressivement l'agitation jusqu'à 30°C.

### • Observations au microscope

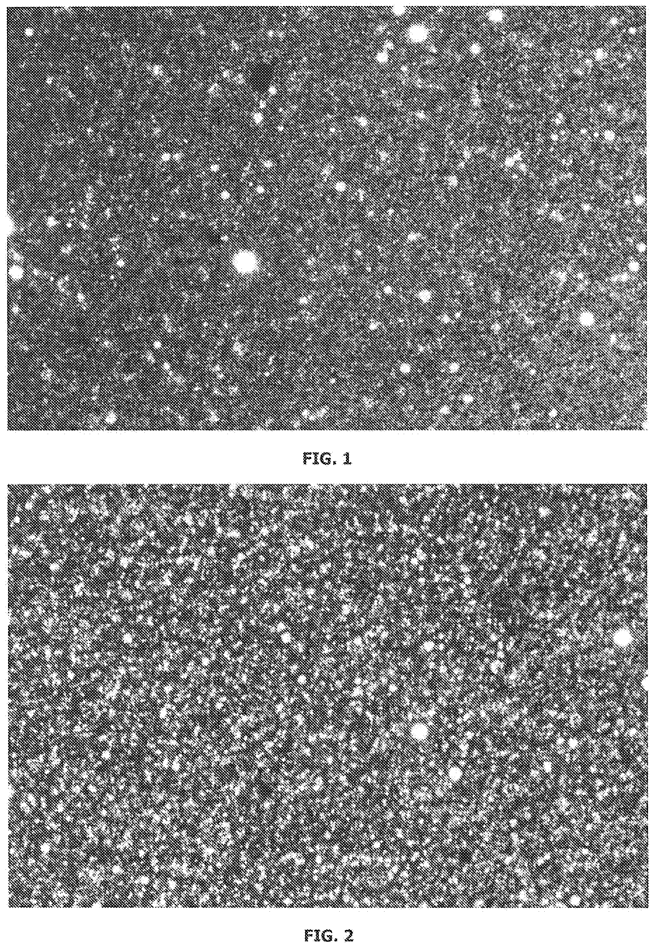

On a observé les compositions de l'Exemple 1 et de l'Exemple 2 comparatif au microscope à un grossissement de 100. La Figure 1 et la Figure 2 présentent les clichés obtenus.

Les dispersions des deux compositions sont homogènes, régulières et assez serrées ; les cires et les pigments sont bien dispersés.

L'ester de THPE permet d'obtenir des dispersions aussi fines que celles obtenues avec l'ester de triéthanolamine.

### • Mesure de la viscosité et du pH

### Méthode de mesure

Chaque formule a été conditionnée dans un pilulier de 120mL et laissée au repos 24h à l'étuve à une température de 25°C
Appareil : Rhéolab QC
Mobile de mesure : ST22-2V
Rotation 50 tr/min pendant 7min

### Résultats

Les valeurs mesurées sont présentées dans le tableau 2 ci-dessous.

La viscosité du mascara de l'invention est de l'ordre de 150.000 à 160.000 mPa.s, tandis que celle du mascara contenant du stéarate de triéthanolamine est de l'ordre de 200.000 mPa.s.

**TABLEAU 2 - Caractérisation physico-chimique**

| | Exemple 1 | Exemple 2 comparatif | Exemple 3 comparatif |
|---|---|---|---|
| VISCOSITE (24h) CPS | 156900 | 206700 | 181400 |
| pH (24h) | 8,61 | 8,45 | 8,94 |

### • Résultat maquillage

Les résultats sont présentés dans le Tableau 3 ci-dessous.

**TABLEAU 3 - Maquillage et qualités d'application**

| | Exemple 1 | Exemple 2 comparatif | Exemple 3 comparatif |
|---|---|---|---|
| CREMEUX | + | ++ | - |
| VOLUME | ++ | ++ | + |
| SOUPLESSE DES CILS | + | + | - |
| ADHERENCE | +++ | ++ | - |

| | | |
|---|---|---|
| Légende : | - | insuffisant |
| | + | résultat médian |
| | ++ | très bon |
| | +++ | excellent |

La composition de l'invention est plus fluide, plus adhérente que les témoins de l'art antérieur contenant du stéarate et du palmitate de triéthanolamine, si bien que les cils sont maquillés en un seul passage de brosse. Le mascara est plus agréable à appliquer et la teinte noire perçue est plus profonde. Les quantités de produit déposées sur les cils sont équivalentes pour l'une et l'autre des compositions.

### Exemple 4, Exemple 5 comparatif et Exemple 6 comparatif :

Trois formules de mascaras ont été préparées en utilisant les ingrédients dans les proportions indiquées dans le Tableau 4 ci-dessous.

**TABLEAU 4 - Composition des mascaras**

| Phase | Ingrédients (Dénomination INCI) | Exemple 4 | Exemple 5 comparatif | Exemple 6 comparatif |
|---|---|---|---|---|
| A1 | WATER | Qsp 100 | Qsp 100 | Qsp 100 |
| A1 | PRESERVATIVES | 1,20 | 1,20 | 1,20 |
| A1 | TRIETHANOLAMINE | - | **2,25** | - |
| A1 | AMINOMETHYL PROPANEDIOL (AMPD ULTRA PC) | - | - | **1,59** |
| A1 | TETRAHYDROXYPROPYL ETHYLENEDIAMINE (ADEKA CARPOL MD-100) | **4,42** | - | - |
| A2 | ACACIA SENEGAL GUM | 1,70 | 1,70 | 1,70 |
| A2 | HYDROXYETHYLCELLULOSE | 1,00 | 1,00 | 1,00 |
| B1 | STEARIC ACID | 2,25 | 2,25 | 2,25 |
| B1 | PALMITIC ACID | 2,25 | 2,25 | 2,25 |
| B1 | PARAFFINE | 10,90 | 10,90 | 10,90 |
| B1 | VP/EICOSENE COPOLYMER (ANTARON V 220 F) | 1,90 | 1,90 | 1,90 |
| B1 | BIS DIGLYCERYL POLYACYLADIPATE-2 (SOFTISAN 649 MB) | 2,80 | 2,80 | 2,80 |
| B1 | POLYBUTENE (POLYBUTENE M 2000) | 5,60 | 5,60 | 5,60 |
| B1 | CANDELLILA CERA | 0,90 | 0,90 | 0,90 |
| B1 | BEESWAX | 6,50 | 6,50 | 6,50 |
| B1 | CERA CARNAUBA | 1,30 | 1,30 | 1,30 |
| B2 | BLACK IRON OXIDE | 8,00 | 8,00 | 8,00 |

Le procédé de préparation est le même que celui décrit dans les Exemples précédents.

### • Mesure de la viscosité et du pH

### Méthode de mesure

Elle est identique à celle décrite précédemment

### Résultats

Les valeurs mesurées sont présentées dans le tableau 5 ci-dessous.

La viscosité du mascara de l'invention est de l'ordre de 143.000 mPa.s tandis que celle du mascara contenant du stéarate de triéthanolamine est de l'ordre de 165.000 mPa.s.

**TABLEAU 5 - Caractérisation physico-chimique**

| | Exemple 4 | Exemple 5 comparatif | Exemple 6 comparatif |
|---|---|---|---|
| VISCOSITE (24h) CPS | 143000 | 165000 | 200900 |
| pH (24h) | 8,29 | 7,92 | 8,59 |

### • Résultat maquillage

Les résultats sont présentés dans le tableau 6 ci-dessous.

**TABLEAU 6 - Maquillage et qualités d'application**

| | Exemple 4 | Exemple 5 comparatif | Exemple 6 comparatif |
|---|---|---|---|
| CREMEUX | ++ | +++ | - |
| VOLUME | ++ | ++ | + |
| SOUPLESSE DES CILS | ++ | ++ | + |
| ADHERENCE | +++ | ++ | + |

| | | |
|---|---|---|
| Légende : | - | insuffisant |
| | + | résultat médian |
| | ++ | très bon |
| | +++ | excellent |

### • Dosage des nitrosamines

### Méthode de mesure

Les deux formules sont conditionnées dans un pilulier de 120mL et laissées au repos 6 mois à l'étuve à une température de 40°C, puis le dosage est effectué selon la norme ISO 15819:2014.

### Résultats

Les valeurs mesurées sont présentées dans le tableau 7 ci-dessous.

**TABLEAU 7 - Dosage des nitrosamines**

| Conditions | Exemple 4 | Exemple 5 comparatif | Exemple 6 comparatif |
|---|---|---|---|
| 6 mois à 20°C | < 10 ppb | 19 ppb | 65 ppb |
| 6 mois à 40°C | < 10 ppb | 237 ppb | < 10 ppb |

La teneur en NDLEA est conforme (soit inférieure à 10 ppb à 20°C et 40°C) au bout de 6 mois de conservation pour la formule de l'invention, tandis qu'elle est supérieure à 20 ppb pour la formule comparative contenant du stéarate de triethanolamine.

## Revendications

1. Composition cosmétique de soin ou de maquillage des cils ou des sourcils comprenant des cires en émulsion dans une phase aqueuse et au moins un ester obtenu par réaction d'un acide gras linéaire et saturé comprenant de 16 à 20 atomes de carbone, et de la tétrahydroxypropyl éthylène diamine, le ratio molaire entre l'acide gras et l'amine étant compris entre 0,9 et 5, l'acide gras représentant de 2,0 à 8,0% en masse et l'amine représentant de 3,0 à 8,0% en masse, par rapport à la masse de la composition.

2. Composition selon la revendication 1, **caractérisée en ce que** l'acide gras est l'acide palmitique, l'acide stéarique ou l'acide béhénique.

3. Composition selon la revendication 1, **caractérisée en ce qu'**elle comprend le mélange d'un ester de l'acide stéarique et de la tétrahydroxypropyl éthylène diamine, et d'un ester de l'acide palmitique et de la tétrahydroxypropyl éthylène diamine, les deux esters étant de préférence en quantités massiques égales.

4. Composition selon l'une des revendications précédentes, **caractérisée en ce que** les cires représentent de 10 à 35% en masse.

5. Composition selon l'une des revendications précédentes, **caractérisée en ce que** le mélange d'au moins une cire dont le point de fusion est supérieur ou égal à 70°C et d'au moins une cire dont le point de fusion est inférieur à 70°C, l'une des cires étant une cire alcane et l'autre étant une cire polaire.

6. Composition selon l'une des revendications précédentes, **caractérisée en ce que** sa viscosité est comprise entre 140 000 mPa.s et 210 000 mPa.s, mesurée à 25°C à l'aide d'un Rhéomat^{®} 180 (société LAMY).

7. Composition selon l'une des revendications précédentes, **caractérisée en ce qu'**elle comprend au moins un tensio-actif non ionique dont le HLB est inférieur à 8 tel que le stéarate de glycéryle ou le composé de dénomination INCI STEARETH-2.

8. Composition selon l'une des revendications précédentes, **caractérisée en ce qu'**elle comprend au moins un tensio-actif non ionique de HLB supérieur à 8 tel que le composé de dénomination INCI STERAETH-20.

9. Composition selon l'une des revendications précédentes, **caractérisée en ce qu'**elle contient entre 40 et 55% en masse d'eau.

10. Procédé de maquillage des cils ou des sourcils qui consiste à appliquer sur les cils ou les sourcils une composition selon l'une des revendications précédentes.

## Patentansprüche

1. Kosmetische Zusammensetzung zur Pflege oder zum Schminken der Wimpern oder Augenbrauen, die Wachse in Emulsion in einer wässrigen Phase und mindestens einen Ester enthält, der durch Reaktion einer linearen und gesättigten Fettsäure mit 16 bis 20 Kohlenstoffatomen erhalten wird, und Tetrahydroxypropylethylendiamin, wobei das Molverhältnis zwischen der Fettsäure und dem Amin zwischen 0,9 und 5 liegt, wobei die Fettsäure 2,0 bis 8,0 Ma% und das Amin 0,3 bis 8,0 Ma%, bezogen auf die Masse der Zusammensetzung, darstellt.

2. Zusammensetzung nach Anspruch 1, **dadurch gekennzeichnet, dass** die Fettsäure Palmitinsäure, Stearinsäure oder Behensäure ist.

3. Zusammensetzung nach Anspruch 1, **dadurch gekennzeichnet, dass** sie die Mischung eines Esters der Stearinsäure und des Tetrahydroxypropylethylendiamins und eines Esters der Palmitinsäure und des Tetrahydroxypropylethylendiamins umfasst, wobei die beiden Ester vorzugsweise in gleichen Massenanteilen vorliegen.

4. Zusammensetzung nach einem der vorstehenden Ansprüche, **dadurch gekennzeichnet, dass** die Wachse 10 bis 35 Ma% darstellen.

5. Zusammensetzung nach einem der vorstehenden Ansprüche, **dadurch gekennzeichnet, dass** die Mischung aus mindestens einem Wachs mit einem Schmelzpunkt von über oder gleich 70 °C und aus mindestens einem Wachs mit einem Schmelzpunkt von unter 70 °C, wobei eines der Wachse ein Alkanwachs und das andere ein polares Wachs ist.

6. Zusammensetzung nach einem der vorstehenden Ansprüche, **dadurch gekennzeichnet, dass** ihre Viskosität zwischen 140.000 mPa.s und 210.000 mPa.s liegt, gemessen bei 25 °C mit einem Rheometer vom Typ "^{®} 180" (Firma LAMY).

7. Zusammensetzung nach einem der vorstehenden Ansprüche, **dadurch gekennzeichnet, dass** sie mindestens ein nichtionisches Tensid mit einem HLB-Wert von unter 8 wie Glycerylstearat oder die Verbindung mit der INCI-Bezeichnung STEARETH-2 enthält.

8. Zusammensetzung nach einem der vorstehenden Ansprüche, **dadurch gekennzeichnet, dass** sie mindestens ein nichtionisches Tensid mit einem HLB-Wert von über 8 wie die Verbindung mit der INCI-Bezeichnung STERAETH-20 enthält.

9. Zusammensetzung nach einem der vorstehenden Ansprüche, **dadurch gekennzeichnet, dass** sie zwischen 40 und 55 Ma% Wasser enthält.

10. Verfahren zum Schminken der Wimpern oder Augenbrauen, das darin besteht, eine Zusammensetzung nach einem der vorstehenden Ansprüche auf die Wimpern oder Augenbrauen aufzutragen.

## Claims

1. A cosmetic composition for caring for or making up the eyelashes or the eyebrows, comprising waxes in emulsion in an aqueous phase and at least one ester obtained by reacting a linear and saturated fatty acid comprising from 16 to 20 carbon atoms, and tetrahydroxypropyl ethylenediamine, the mole ratio between the fatty acid and the amine being between 0.9 and 5, the fatty acid representing from 2.0 to 8.0% by weight and the amine representing from 3.0 to 8.0% by weight, relative to the weight of the composition.

2. The composition as claimed in claim 1, **characterized in that** the fatty acid is palmitic acid, stearic acid or behenic acid.

3. The composition as claimed in claim 1, **characterized in that** it comprises the mixture of an ester of stearic acid and of tetrahydroxypropyl ethylenediamine, and an ester of palmitic acid and of tetrahydroxypropyl ethylenediamine, the two esters preferably being in equal weight amounts.

4. The composition as claimed in one of the preceding claims, **characterized in that** the waxes represent from 10 to 35% by weight.

5. The composition as claimed in one of the preceding claims, **characterized in that** the mixture of at least one wax, the melting point of which is greater than or equal to 70°C, and of at least one wax, the melting point of which is less than 70°C, one of the waxes being an alkane wax and the other being a polar wax.

6. The composition as claimed in one of the preceding claims, **characterized in that** its viscosity is between 140 000 mPa.s and 210 000 mPa.s, measured at 25°C using a Rheomat^{®} 180 (LAMY company).

7. The composition as claimed in one of the preceding claims, **characterized in that** it comprises at least one nonionic surfactant, the HLB of which is less than 8, such as glyceryl stearate or the compound with the INCI name Steareth-2.

8. The composition as claimed in one of the preceding claims, **characterized in that** it comprises at least one nonionic surfactant having an HLB greater than 8, such as the compound with the INCI name Steareth-20.

9. The composition as claimed in one of the preceding claims, **characterized in that** it contains between 40 and 55% by weight of water.

10. A process for making up the eyelashes or the eyebrows which consists in applying to the eyelashes or the eyebrows a composition as claimed in one of the preceding claims.
